# EUROPEAN PATENT SPECIFICATION

(11) **EP 2 863 815 B1**
(45) Date of publication and mention of the grant of the patent: **22.02.2017**
(21) Application number: 13807560.1
(22) Date of filing: 23.05.2013
(51) Int. Cl.: A61B 17/34

(54) **ACCESS PORT**
ZUGANGSPORT
ORIFICE D'ACCÈS

(30) Priority: 21.06.2012 JP 2012140081
(43) Date of publication of application: 29.04.2015
(73) Proprietor: Olympus Corporation, Tokyo 192-8507 (JP)
(72) Inventor: KOBAYASHI, Masayuki, Tokyo 192-8507 (JP)
(74) Representative: Schicker, Silvia
(86) International application number: PCT/JP2013/064964
(87) International publication number: WO 2013/190967

(56) References cited:
- EP-A1- 0 689 467
- EP-A1- 1 862 128
- WO-A1-86/05378
- WO-A1-96/25886
- WO-A1-2007/019137
- WO-A1-2010/098905
- WO-A1-2011/130456
- JP-A- H05 317 325
- JP-A- H07 265 327
- US-A1- 2008 009 891
- US-A1- 2010 105 981
- US-A1- 2010 105 981

## Description

### {Technical Field}

The present invention relates to an access port.

### {Background Art}

A known gastric fistula catheter or trocar in the related art is equipped, at a distal end thereof, with a balloon or a superelastic expanding member that is expandable in a radial direction and that is designed to prevent the catheter or trocar from coming out by expanding the balloon or the superelastic expanding member, in a state in which it penetrates a tissue wall so that the distal end is inserted in a body. (for example, see PTLs 1 and 2).

### {Citation List}

### {Patent Literature}

{PTL 1} Japanese Unexamined Patent Application, Publication No. 2010-158486
{PTL 2} Japanese Unexamined Patent Application, Publication No. Hei 9-28666

WO 2011/130456 A1 discloses a device for accessing the pericardial space of a heart. The access device comprises a tissue-piercing member, a first guide element, a second guide element, a sheath, a tissue-piercing member actuator, a sheath actuator and a tissue-engaging member. Movement of the tissue-piercing member is controlled by the tissue-piercing member actuator which is positioned at the proximal end of the tissue-piercing member.

US 2010/0105981 A1, upon which the preamble of claim 1 is based, discloses a puncturing instrument which comprises a guiding tube at the distal end of which an expaning anchor is integrally formed. The puncturing instrument further comprises an anchor or protective sleeve which encloses at least a distal end portion of the guiding tube especially in the area of the expanding anchor in a manner to be axially displaceable.

### {Summary of Invention}

### {Technical Problem}

However, the apparatuses disclosed in PTLs 1 and 2 constitute a retaining mechanism by using a member having elasticity, such as a balloon or a superelastic expanding member. Therefore, they have a disadvantage in that, although they function effectively for a relatively thick tissue wall, they do not function as an effective retaining mechanism for a thin, highly stretchable biological membrane, such as a pericardium.

The present invention is made in consideration of the above circumstances, and an object thereof is to provide an access port that can be fixed to a biological membrane, such as the pericardium, in a state in which it penetrates the biological membrane.

### {Solution to Problem}

To achieve the above object, the present invention provides an access port as defined in claim 1.

An aspect provides an access port comprising a tubular member having a through-hole passing therethrough in an axial direction; a needle-like member having a tip pointing toward a rear end of the tubular member and mounted to an outer surface of the tubular member; and a manipulating means for switching between a state in which the tip is projected and a state in which the tip is not projected.

Furthermore, in the above aspect, the manipulating means may switch between a state in which the tip is projected radially outwards from the outer surface of the tubular member and a state in which the tip is not projected therefrom.

According to this aspect, by bringing the distal end of the needle-like member to a state in which it is not projected from the outer surface of the tubular member with the manipulating means, inserting the distal end portion of the tubular member to a position at which the needle-like member passes through the biological membrane in the hole formed to penetrate the biological membrane, bringing the tip of the needle-like member into a state in which it is projected radially outwards from the outer surface of the tubular member with the manipulating means, and moving the tubular member in a direction in which it is withdrawn from the biological membrane, the tip of the needle-like member projecting radially outwards passes through the biological membrane, and the needle-like member is hooked to the biological membrane and is fixed thereto so as not to come off. This allows an endoscope, a treatment tool, or the like to be introduced from outside of the biological membrane to inside of the biological membrane through the through-hole in the tubular member.

To remove the tubular member, the engagement of the needle-like member with the biological membrane is released by moving the tubular member toward the distal end, and thereafter, the tip of the needle-like member is moved by the manipulating means from the position radially outside the tubular member to a position at which the tip is not projected. Thereafter, by moving the tubular member toward the rear end, the tubular member is withdrawn from the hole in the biological membrane without the needle-like member being hooked to the biological membrane. This allows the access port to be removed without damaging the biological membrane.

In the above aspect, a plurality of the needle-like members may be provided at intervals in a circumferential direction of the tubular member.

This allows the needle-like member to be hooked to the biological membrane at the plurality of locations at intervals in the circumferential direction of the tubular member, thereby stably fixing the tubular member to the biological membrane.

Furthermore, in the above aspect, a recess accommodating the tip in a state in which the tip is not projected radially outwards from the outer surface of the tubular member is provided in the outer surface of the tubular member.

This allows the tip to be accommodated in the recess by manipulating the manipulating means to facilitate insertion of the access port into the biological membrane and removal from the interior of the biological membrane and allows the tip to be stuck into the biological membrane to hook it thereto by extracting the tip from the recess and making it project radially outwards.

Furthermore, in the above aspect, the needle-like member may include a rotary shaft extending in the axial direction and a needle portion provided by bending a distal end of the rotary shaft and having the tip at the distal end; and the manipulating means may include a handle provided at a rear end of the rotary shaft and causing the rotary shaft to rotate about the axis thereof.

This can facilitate insertion of the tip of the needle portion into the biological membrane and removal from the interior of the biological membrane, and allows the tip to be fixed to the biological membrane by sticking it therein, by rotating the rotary shaft by manipulating the handle to switch the tip position of the needle portion, formed by bending the tip of the rotary shaft, between a projecting radially outward position and a retracted position.

Furthermore, in the above aspect, a level-difference recessed radially inwards may be provided in the outer surface of the tubular member; the needle-like member may be disposed at a shoulder portion of the level-difference; and the manipulating means may be a cap that can move between a position at which the cap covers the tip after the cap approaches the tip from a rear of the tip and a position at which the tip is exposed.

This can facilitate insertion of the tip of the needle-like member into the biological membrane and removal from the biological membrane by moving the cap to cover the tip of the needle-like member and make it easier to stick the tip into the biological membrane by exposing the tip and making the tip project in the radially outward direction of the tubular member.

Furthermore, in the above aspect, a recess recessed radially inwards may be provided in the outer surface of the tubular member; the needle-like member may be disposed on an inner surface of the recess; and the manipulating means may include an object that is inserted in a gap between the needle-like member and a bottom surface of the recess, and the inserted object thicker than the gap moves along a longitudinal direction of the needle-like member.

This allows the needle-like member to be flexibly deformed merely by moving the inserted object with the manipulating means to switch between a state in which it is projected radially outwards from the outer surface of the tubular member and a state in which it is retracted radially inward retracted from the outer surface of the tubular member.

In the above aspect, the needle-like member may be provided such that the tip is exposed outside the tubular member and so as to be swiveled about a hinge shaft extending in a tangential direction of the tubular member; and the manipulating means may switch the tip to a state in which the tip is not projected by pushing a base end of the needle-like member radially outwards from the through-hole side.

By doing so, when an inserted member, such as a dilator, is inserted into the through-hole of the tubular member, the base end of the needle-like member can be pushed radially outwards from the through-hole side to cause the needle-like member to swivel about the hinge shaft, thus allowing the tip to be brought into a state in which it is not projected radially outwards from the outer surface of the tubular member. This can facilitate insertion of the tubular member into the biological membrane and removal of the tubular member from the interior of the biological membrane. On the other hand, by removing the inserted member from the through-hole, the needle-like member is made to swivel so that the base end thereof is projected into the through-hole, and the tip can be brought into a state in which it is projected radially outwards from the outer surface of the tubular member.

### {Advantageous Effects of Invention}

The present invention provides an advantage in that an access port can be fixed to a biological membrane, such as the pericardium, in a state in which it penetrates the biological membrane.

### {Brief Description of Drawings}

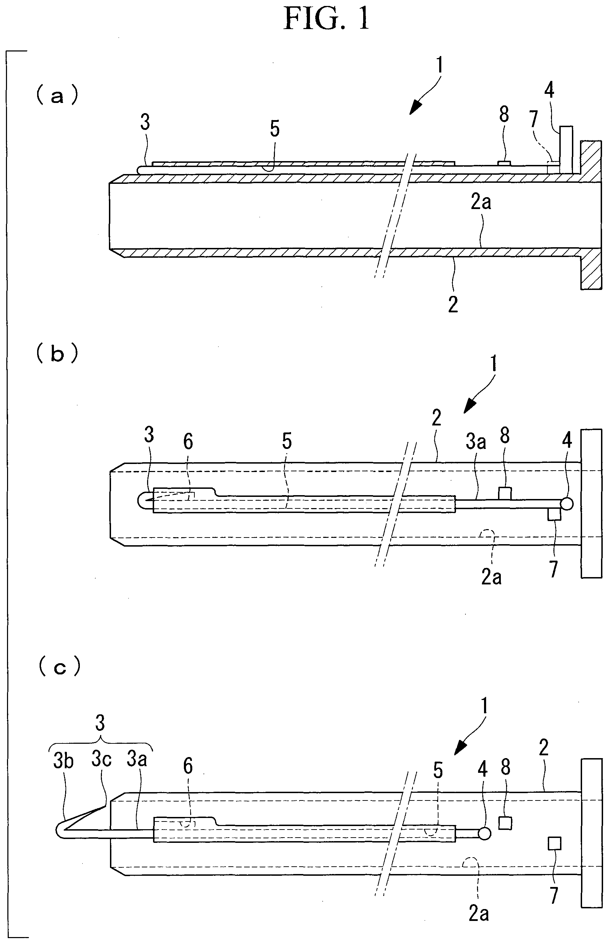

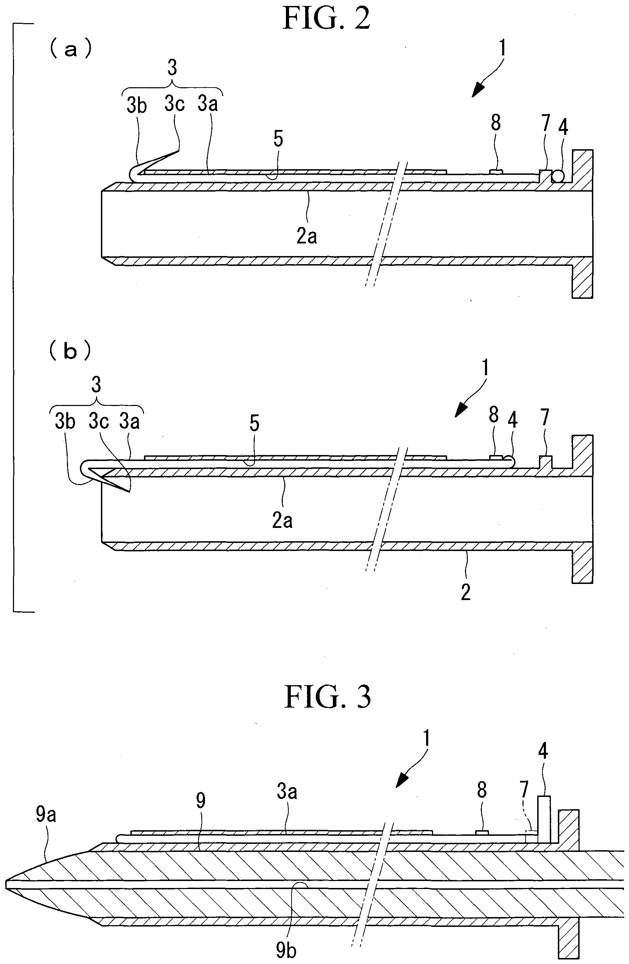

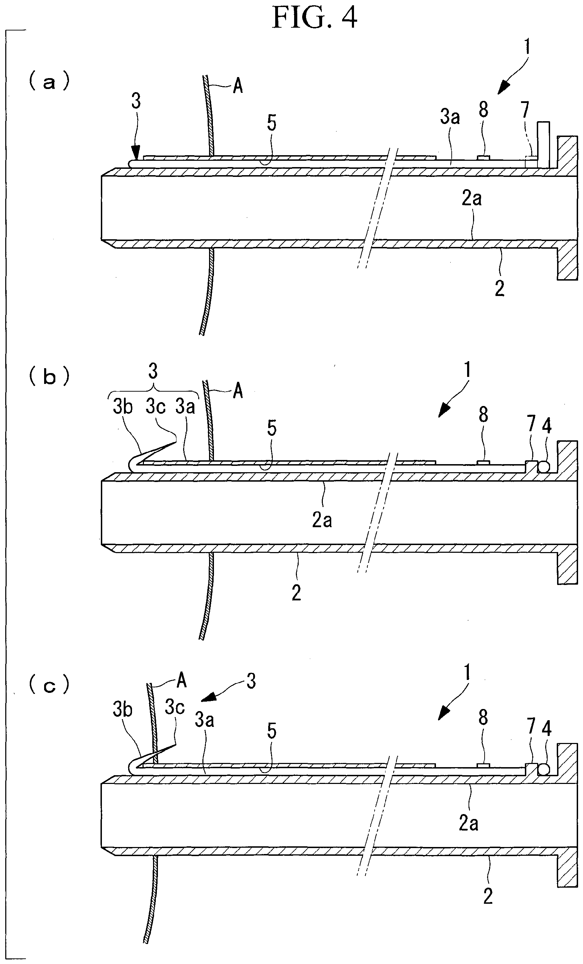

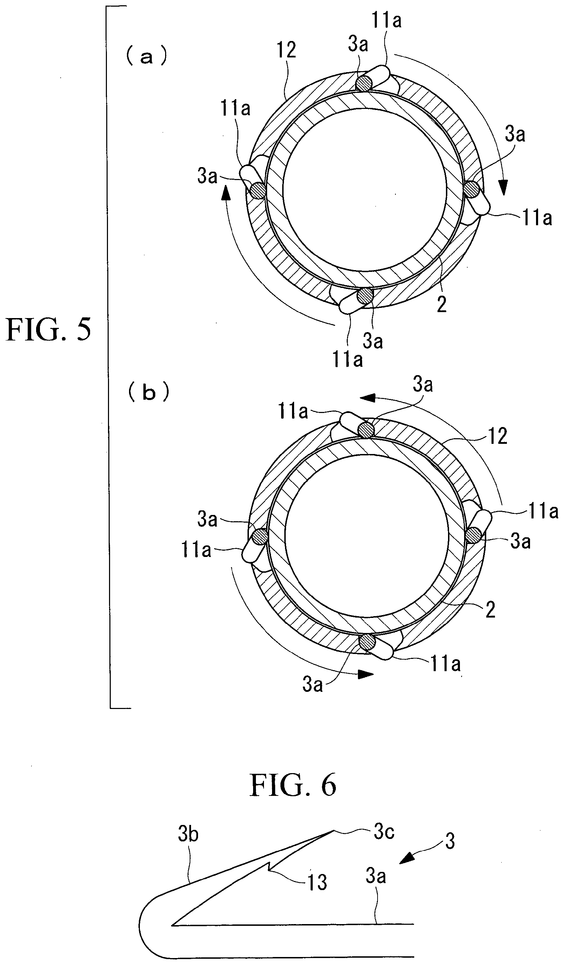

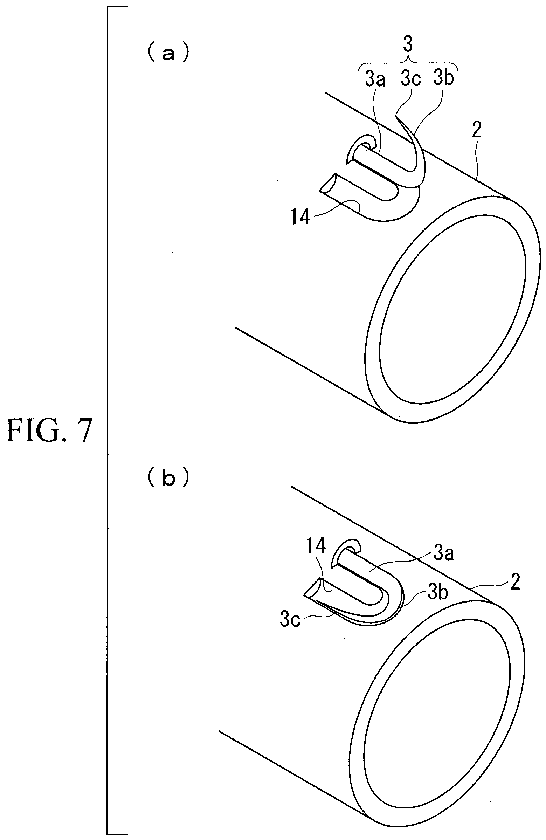

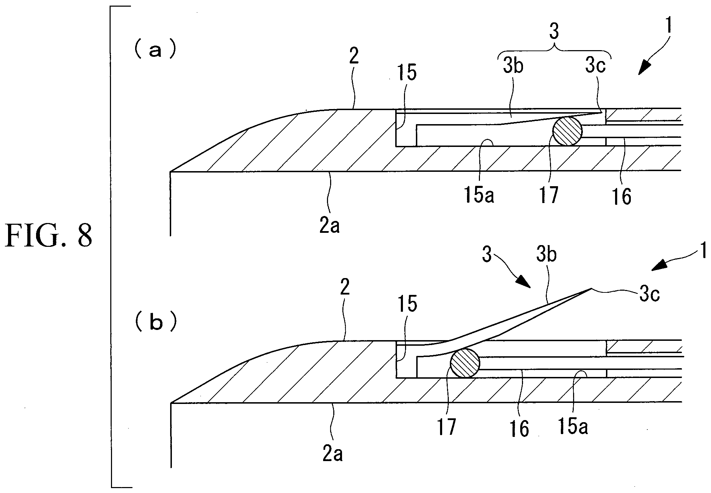

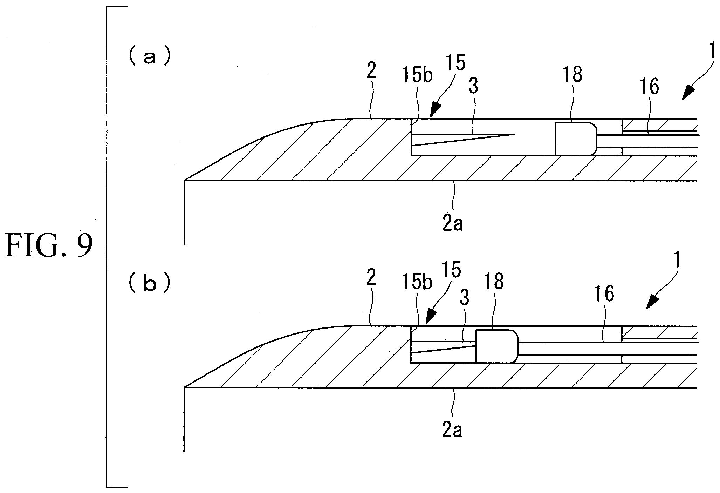

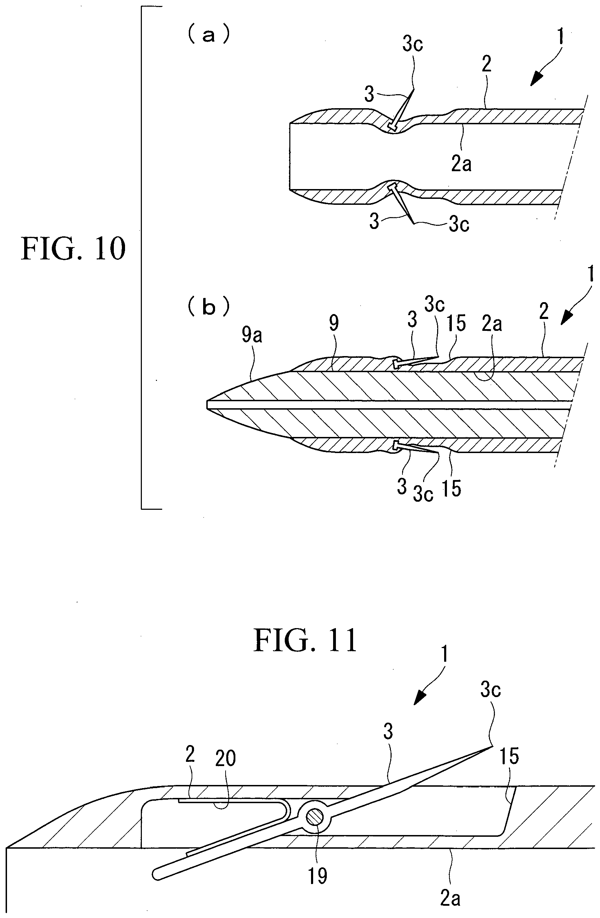

{Fig. 1} Fig. 1 is a diagram of an access port according to an embodiment of the present invention, in which (a) is a longitudinal-sectional view showing a state in which a needle-like member is accommodated, (b) is a plan view of the state in (a), and (c) is a plan view of a state in which the needle-like member is released, respectively.
{Fig. 2} Fig. 2 is a longitudinal-sectional view of the access port in Fig. 1, in which (a) shows a state in which the needle-like member is disposed pointing radially outwards, and (b) shows a state in which it is disposed pointing radially inwards, respectively.
{Fig. 3} Fig. 3 is a longitudinal-sectional view of the access port in Fig. 1, showing a state in which a dilator is inserted into a central through-hole.
{Fig. 4} Fig. 4 is a longitudinal-sectional view of the access port in Fig. 1, in which (a) shows a state in which it is inserted into a pericardium, (b) shows a state in which the needle-like member is placed in position, and (c) shows a state in which the tip of the needle-like member penetrates the pericardium and is hooked thereto, respectively.
{Fig. 5} Fig. 5 is a cross-sectional view of a first modification of the access port in Fig. 1, in which (a) shows a state in which a manipulating ring is rotated in one direction in the case where a plurality of needle-like members are provided, and (b) shows a state in which it is rotated in the other direction, respectively.
{Fig. 6} Fig. 6 is an enlarged view of a needle-like member of a second modification of the access port in Fig. 1, in which the needle-like member has a barb in the vicinity of the tip.
{Fig. 7} Fig. 7 is a perspective view of a third modification of the access port in Fig. 1, in which (a) shows a state in which a needle portion is placed upright from a recess, and (b) shows a state in which the needle portion is accommodated in the recess, respectively.
{Fig. 8} Fig. 8 is a partial enlarged longitudinal-sectional view of a fourth modification of the access port in Fig. 1, in which (a) shows a state in which a flexible needle portion is accommodated in a recess, and (b) shows a state in which the tip is projected from the recess, respectively.
{Fig. 9} Fig. 9 is a partial enlarged longitudinal-sectional view of a fifth modification of the access port in Fig. 1, in which (a) shows a state in which the tip of a needle-like member is exposed, and (b) shows a state in which the tip is covered with a cap, respectively.
{Fig. 10} Fig. 10 is a longitudinal-sectional view of a sixth modification of the access port in Fig. 7, in which (a) shows a state in which needle portions are projected radially outwards, and (b) shows a state in which the needle portions are retracted by inserting a dilator, respectively.
{Fig. 11} Fig. 11 is a partial enlarged longitudinal-sectional view illustrating a swiveling mechanism equivalent to Fig. 10.

### {Description of Embodiments}

An access port 1 will be described hereinbelow with reference to the drawings.

The scope of the invention is as defined in the claims appended hereto.

This embodiment will be described using a pericardium A as an example of a biological membrane to which the access port 1 is to be attached.

As shown in Fig. 1(a), the access port 1 according to this embodiment is equipped with a substantially cylindrical straight-pipe-shaped tubular member 2 having a central through-hole 2a, a needle-like member 3 mounted to the outer surface of the tubular member 2, and a manipulating means 4 for shifting the needle-like member 3.

The tubular member 2 has, in the vicinity of the outer surface thereof, a needle through-hole 5 disposed parallel to the central through-hole 2a.

An accommodating hole 6 that is formed beside the opening of the needle through-hole 5 is provided at the distal end of the needle through-hole 5.

The needle-like member 3 is equipped with a circular-cross-section straight-rod-like rotary shaft portion 3a that is inserted into the needle through-hole 5 of the tubular member 2 so as to be capable of rotating about the axis and a needle portion 3b formed by bending the distal end of the rotary shaft portion 3a so as to form an acute angle to the rotary shaft portion 3a. The needle portion 3b of the needle-like member 3 has a sharp tip 3c at the distal end. The needle-like member 3 is constituted by a metal material having flexibility, and when the tip 3c is to be accommodated in the accommodating hole 6, as shown in Fig. 1(b), the needle portion 3b is brought close to the rotary shaft portion 3a owing to flexible deformation, and when the tip 3c is released from the accommodating hole 6, the needle portion 3b is separated from the rotary shaft portion 3a to a predetermined angle.

Furthermore, the needle-like member 3 is provided such that the rotary shaft portion 3a can move also in the axial direction in the needle through-hole 5.

The manipulating means 4 is constituted by a handle (hereinafter also referred to as a handle 4) configured by bending the rear end (base end) of the rotary shaft portion 3a at a substantially right angle to the rotary shaft portion 3a.

When the handle 4 is pulled down in one direction at substantially 90° in the state in Fig. 1(c), in which the tip 3c is released from the accommodating hole 6, the tip 3c of the needle portion 3b is disposed so as to project radially outwards from the outer surface of the tubular member 2, as shown in Fig. 2(a). On the other hand, when the handle 4 is pulled down in the other direction at substantially 90°, the tip 3c of the needle portion 3b is disposed in the central through-hole 2a of the tubular member 2, as shown in Fig. 2 (b) .

Furthermore, a projection 7 that secures the handle 4 when the tip 3c of the needle portion 3b is pointed radially outwards, as shown in Fig. 2(a), and a projection 8 that secures the handle 4 when the tip 3c of the needle portion 3b is pointed radially inwards, as shown in Fig. 2(b), are provided at the outer surface of the tubular member 2 in the vicinity of the handle 4.

The operation of the thus-configured access port 1 according to this embodiment will be described hereinbelow.

To fix the access port 1 according to this embodiment in the state in which it penetrates the pericardium A, first, a puncture needle (not shown) is passed through the pericardium A, and a guide wire (not shown) is set in place using the puncture needle. The puncture needle is then removed so that only the guide wire remains in place, penetrating the pericardium A.

In this state, as shown in Fig. 3, a dilator 9 is snugly fitted into the central through-hole 2a of the tubular member 2 of the access port 1 according to this embodiment. At this time, the needle portion 3b of the needle-like member 3 has been flexibly deformed so as to be brought close to the rotary shaft portion 3a, and the tip 3c thereof is accommodated in the accommodating hole 6.

The dilator 9 has, at the distal end, a tapered portion 9a that smoothly connects to the distal end of the tubular member 2 and a through-hole 9b having a diameter that allows the guide wire to be passed therethrough. With the guide wire passed through the through-hole 9b, the assembly of the dilator 9 and the access port 1 is moved forward along the guide wire. Thus, the tissue is pushed open by the tapered portion 9a at the distal end of the dilator 9, and the assembly moves forward, and furthermore, the hole formed in the pericardium A is increased in diameter by the tapered portion.

Then, once the assembly has been moved forward until the distal end portion of the needle-like member 3 provided in the tubular member 2 completely enters the pericardium A, as shown in Fig. 4(a), the dilator 9 and the guide wire are removed.

In this state, the handle 4 is manipulated to move the needle-like member 3 forwards relative to the tubular member 2, as shown in Fig. 1(c). This causes the tip 3c accommodated in the accommodating hole 6 to come out from inside the accommodating hole 6, causing the flexibly deformed needle portion 3b to separate from the rotary shaft portion 3a due to the spring force, thus forming the hook-shaped needle-like member 3.

By rotating the handle 4 by substantially 90° counterclockwise, looking toward the distal end, while moving it backwards, this state shifts to a state shown in Fig. 4(b), and thus, the handle 4 is secured by being hooked on the projection 7.

Thus, by moving back the tubular member 2 and the needle-like member 3, which are integrally fixed to each other, together relative to the pericardium A, the tip 3c of the needle portion 3b projecting radially outwards is stuck into the pericardium A from the interior to hook it, as shown in Fig. 4(c).

As described above, the access port 1 according to this embodiment has an advantage in that even if a force that pulls the tubular member 2 outside the pericardium A is exerted on the tubular member 2, the tubular member 2 is reliably prevented from being withdrawn because the needle portion 3b is hooked to the pericardium A.

In this state, an endoscope or a treatment tool can be introduced into the pericardium A through the central through-hole 2a of the tubular member 2 to perform a treatment on the heart.

In addition, to extract the tubular member 2 from the body upon completion of the procedure, the tubular member 2 and the needle-like member 3 are moved forwards relative to the pericardium A to return to a state in which the needle portion 3b is not hooked to the pericardium A, as shown in Fig. 4(b), to disengage the handle 4 and the projection 7, and the handle 4 is moved forwards relative to the tubular member 2 into the state shown in Fig. 1(c). By pointing the needle portion 3b inwards in the radial direction of the tubular member 2 by rotating the handle 4 clockwise, as looking toward the distal end, and thereafter slightly moving the needle-like member 3 backwards relative to the tubular member 2, the needle portion 3b is hooked on the distal end of the tubular member 2, as shown in Fig. 2(b). By engaging the handle 4 with the projection 8, the needle-like member 3 is fixed to the tubular member 2 at that position.

Since this brings the needle-like member 3 into a state in which it is not projected radially outwards from the outer surface of the tubular member 2, the tubular member 2 and the needle-like member 3 can easily be withdrawn from the body without the needle portion 3b being hooked to the pericardium A by retracting the tubular member 2 and the needle-like member 3 together.

Although this embodiment has been described as applied to a case in which the tubular member 2 is equipped with a single needle-like member 3, the present invention is not limited thereto; a plurality of needle-like members may be provided at intervals in the circumferential direction. In this case, although four rotary shaft portions 3a may be manipulated with separate handles 4, a manipulating ring 12 that changes the angles of manipulating levers 11a mounted to the four rotary shaft portion 3a at the same time may be provided, as shown in Figs. 5(a) and (b).

In the example shown in Fig. 5, the angles of the four manipulating levers 11a are changed by 90° by rotating the manipulating ring 12 in the circumferential direction, thereby causing the rotary shaft portions 3a and the needle portions 3b at the distal ends thereof to be rotated by 90°.

The rotation by 90° allows the needle portions 3b disposed at positions parallel to the outer surface of the tubular member 2 to be placed upright so as to point radially outwards. Furthermore, the needle portions 3b pointed radially inwards at an angle of 45° can be pointed radially outwards at an angle of 45°.

Providing a plurality of needle-like members 3 at intervals in the circumferential direction has an advantage that the tubular member 2 can be fixed to the pericardium A at a plurality of locations in the circumferential direction, thus allowing retention more reliably.

Furthermore, as shown in Fig. 6, a barb 13 may be provided in the vicinity of the tip 3c of the needle portion 3b. This can make it even more difficult for the penetrated pericardium A to come out by using the barb.

Furthermore, as shown in Fig. 7, a recess 14 that accommodates the needle portion 3b may be provided in the outer surface of the tubular member 2.

Specifically, when the tubular member 2 is to be fixed to the pericardium A, the needle-like member 3 is rotated about the axis of the rotary shaft portion 3a to place the needle portion 3b upright, as shown in Fig. 7(a), and when the tubular member 2 is to be inserted into or removed from the pericardium A, the needle portion 3b can be accommodated in the recess 14, as shown in Fig. 7(b), so that the tip 3c does not obstruct insertion or removal.

Furthermore, providing such a recess 14 has an advantage of retention more reliably because, when the needle-like member 3 is rotated so as to be accommodated in the recess 14, with the needle-like member 3 hooked to the pericardium A, the pericardium A is also pulled into the recess 14.

Furthermore, it is also possible that, by fixing the flexible needle-like member 3 in a recess 15 provided in the outer surface of the tubular member 2, with the tip 3c pointed toward the rear end, and by inserting an inserted object 17, which is provided at the distal end of a rod 16 connecting to the handle 4, between a bottom surface 15a of the recess 15 and the needle-like member 3, as shown in Fig. 8(a), the needle-like member 3 is placed upright so that the tip 3c points in the radially outward direction of the tubular member 2, as shown in Fig. 8(b). When the tubular member 2 is to be inserted into or removed from the pericardium A, the state shown in Fig. 8(a) should be used, and when the needle-like member 3 is to be hooked and fixed in the pericardium A, the state shown in Fig. 8(b) should be used.

Furthermore, as shown in Fig. 9(a), the needle-like member 3 whose tip 3c points toward the rear end may be fixed in (a shoulder portion 15b of) the recess (level-difference) 15 provided in the outer surface of the tubular member 2, and a cap 18 provided at the distal end of the rod 16 connecting to the handle 4 may be brought close to or away from the tip 3c of the needle-like member 3 in the longitudinal direction. The cap 18 may be formed of a soft material, such as silicone rubber.

By doing so, when the tubular member 2 is to be inserted into or removed from the pericardium A, the state shown in Fig. 9(b) is used, and the tip 3c of the needle-like member 3 is covered with the cap 18, and when the tubular member 2 is to be fixed in the pericardium A, the state shown in Fig. 9(a) is used, and the tip 3c is exposed so that the tip 3c can be hooked to the pericardium A. Furthermore, covering the needle-like member 3 with the cap 18, with it hooked to the pericardium A, can reliably prevent the pericardium A from coming off the needle-like member 3.

Furthermore, it is also possible that the tubular member 2 be made of an elastic material, and portions in which the needle-like members 3 are embedded be projected radially inwards into the central through-hole 2a, as shown in Fig. 10(a), and in a state in which the dilator 9 is inserted in the central through-hole 2a, as shown in Fig. 10(b), the projecting portions be pushed radially outwards by the dilator 9 so that the needle-like members 3 swivel so as to be parallel to the outer surface of the tubular member 2.

This structure is equivalent to a swiveling mechanism shown in Fig. 11. Specifically, the needle-like members 3 may be provided so as to be swiveled about a hinge shaft 19 disposed along the circumferential direction within the wall thickness of the tubular member 2 and may be configured such that, when the tip 3c of the needle-like member 3 is projected radially outwards from the outer surface of the tubular member 2, the other end of the needle-like member 3 projects into the central through-hole 2a. In the drawing, reference sign 20 denotes a spring.

When the dilator 9 is inserted into the central through-hole 2a, and the projecting end of the needle-like member 3 in the central through-hole 2a is pushed radially outwards by the dilator 9, the needle-like member 3 swivels about the hinge shaft 19, thus allowing the tip 3c to be accommodated in the recess 15 provided in the outer surface of the tubular member 2.

This allows the tubular member 2 to be inserted into or removed from the pericardium A by inserting the dilator 9 into the central through-hole 2a, and the needle-like members 3 to be hooked to the pericardium A by removing the dilator 9 from the central through-hole 2a so that the tips 3c of the needle-like members 3 are projected radially outwards.

Furthermore, although this embodiment shows the pericardium A as an example of a biological membrane, it is not limited thereto; the present invention may be applied to an access port 1 fixed to any other biological membrane in a state in which it penetrates the biological membrane.

### {Reference Signs List}

- 1: access port
- 2: tubular member
- 2a: central through-hole (through-hole)
- 3: needle-like member
- 3a: rotary shaft portion (rotary shaft)
- 3b: needle portion
- 3c: tip
- 4: handle (manipulating means)
- 14: recess
- 15: recess (level-difference)
- 17: inserted object
- 18: cap
- 19: hinge shaft

## Claims

1. An access port (1) comprising:
a tubular member (2) having a through-hole (2a) passing therethrough in an axial direction;
a needle-like member (3) having a tip pointing toward a rear end of the tubular member (2); and
a manipulating means for switching between a state in which the tip (3c) is projected radially outwards from the outer surface of the tubular member (2) and a state in which the tip (3c) is not projected therefrom,
**characterized by** a recess (14) provided in the outer surface of the tubular member (2) and accommodating the tip (3c) in a state in which the tip (3c) is not projected radially outwards from the outer surface of the tubular member (2); and
the needle-like member (3) is mounted to an outer surface of the tubular member (2).

2. The access port according to Claim 1, wherein a plurality of the needle-like members (3) are provided at intervals in a circumferential direction of the tubular member (2).

3. The access port according to Claim 1 to 2, wherein
the needle-like member (3) includes a rotary shaft (3a) extending in the axial direction and a needle portion (3b) provided by bending a distal end of the rotary shaft (3a) and having the tip (3c) at the distal end; and
the manipulating means includes a handle (4) provided at a rear end of the rotary shaft (3a) and causing the rotary shaft (3a) to rotate about the axis thereof.

## Patentansprüche

1. Zugangsanschluss (1), der umfasst
ein rohrförmiges Element (2) mit einer Durchgangsöffnung (2a) die es in einer axialen Richtung durchsetzt;
ein nadelartiges Element (3) mit einer Spitze, die in Richtung eines hinteren Endes des rohrförmigen Elements (2) zeigt; und
ein Manipulationsmittel zum Umschalten zwischen einem Zustand, in dem die Spitze (3c) von der Außenfläche des rohrförmigen Elements (2) radial nach außen vorsteht, und einem Zustand, in dem die Spitze (3c) nicht davon vorsteht,
**gekennzeichnet durch** eine Ausnehmung (14), die in der Außenfläche des rohrförmigen Elements (2) vorgesehen ist und die Spitze (3c) in einem Zustand aufnimmt, in dem die Spitze (3c) nicht radial nach außen von der Außenfläche des rohrförmigen Elements (2) vorsteht; und
**dadurch**, dass das nadelartige Element (3) an einer Außenfläche des rohrförmigen Elements (2) angebracht ist.

2. Zugangsanschluss nach Anspruch 1, wobei eine Mehrzahl von nadelartigen Elementen (3) in Intervallen in einer Umfangsrichtung des rohrförmigen Elements (2) vorgesehen ist.

3. Zugangsanschluss nach Anspruch 1 oder 2, wobei
das nadelartige Element (3) eine Drehwelle (3a), die sich in der axialen Richtung erstreckt, und einen Nadelabschnitt (3b) umfasst, der durch Biegen eines distalen Endes der Drehwelle (3a) erhalten wird und die Spitze (3c) an dem distalen Ende hat; und
das Manipulationsmittel einen Griff (4) umfasst, der an einem hinteren Ende der Drehwelle (3a) vorgesehen ist und die Drehwelle (3a) veranlasst, sich um ihre Achse zu drehen.

## Revendications

1. Port d'accès (1) comprenant :
un élément tubulaire (2) ayant un trou traversant (2a) passant au travers dans une direction axiale ;
un élément de type aiguille (3) ayant une pointe dirigée vers une extrémité arrière de l'élément tubulaire (2) ; et
un moyen de manipulation permettant de passer entre un état dans lequel la pointe (3c) est projetée radialement vers l'extérieur à partir de la surface externe de l'élément tubulaire (2) et un état dans lequel la pointe (3c) n'est pas projetée à partir de celle-ci,
**caractérisé par** un renfoncement (14) prévu dans la surface externe de l'élément tubulaire (2) et logeant la pointe (3c) dans un état dans lequel la pointe (3c) n'est pas projetée radialement vers l'extérieur à partir de la surface externe de l'élément tubulaire (2) ; et
l'élément de type aiguille (3) est monté sur une surface externe de l'élément tubulaire (2).

2. Port d'accès selon la revendication 1, dans lequel une pluralité d'éléments de type aiguille (3) sont prévus à des intervalles dans une direction circonférentielle de l'élément tubulaire (2).

3. Port d'accès selon la revendication 1 ou 2, dans lequel
l'élément de type aiguille (3) comprend un arbre rotatif (3a) s'étendant dans la direction axiale et une partie d'aiguille (3b) prévue par pliage d'une extrémité distale de l'arbre rotatif (3a) et ayant la pointe (3c) au niveau de l'extrémité distale ; et
le moyen de manipulation comprend une poignée (4) prévue au niveau d'une extrémité arrière de l'arbre rotatif (3a) et amenant l'arbre rotatif (3a) à tourner autour de l'axe de celui-ci.
